# FASCICULE DE BREVET EUROPEEN

(11) **EP 4 281 021 B1**
(45) Date de publication et mention de la délivrance du brevet: **18.09.2024**
(21) Numéro de dépôt: 22700938.8
(22) Date de dépôt: 17.01.2022
(51) Int. Cl.: A61F 5/14

(54) **DISPOSITIF ORTHOPEDIQUE DE DECHARGE TALONNIERE**
ORTHOPÄDISCHE VORRICHTUNG ZUR FERSENENTLASTUNG
ORTHOPAEDIC HEEL RELIEF DEVICE

(30) Priorité: 19.01.2021 FR 2100505
(43) Date de publication de la demande: 29.11.2023
(73) Titulaire: Fresnel, Xavier, 74260 Les Gets (FR)
(72) Inventeur: Fresnel, Xavier, 74260 Les Gets (FR)
(74) Mandataire: KATZAROV S.A.
(86) Numéro de dépôt international: PCT/EP2022/050867
(87) Numéro de publication internationale: WO 2022/157111

(56) Documents cités:
- EP-A1- 1 021 102
- WO-A1-94/19978
- FR-A1- 2 871 049
- FR-A1- 2 912 294
- FR-A1- 2 925 290
- FR-A1- 3 031 036

## Description

La présente invention concerne un dispositif orthopédique et une semelle orthopédique. Le document FR 2 871 049 A1décrit un dispositif orthopédique destiné à être fixé à une semelle intérieure d'une chaussure, comprenant un moyen de décharge talonnière, qui comprend une extrémité supérieure destinée à exercer une pression à l'aplomb de la jonction de la partie postérieure de la voûte plantaire et de la partie antérieure du talon, le moyen de décharge talonnière ayant une épaisseur maximale comprise entre 6 et 14 millimètres, le moyen de décharge talonnière comprenant une face inférieure destinée à être fixée à la semelle intérieure, une face supérieure inclinée et une face s'étendant de l'extrémité supérieure de la face supérieure à l'extrémité de la face inférieure agencée en correspondance, la jonction de la face supérieure inclinée et de la face formant une arête correspondant à l'extrémité supérieure du moyen de décharge talonnière.

Le talon est la composante anatomique située à la verticale de la jambe sur l'arrière du pied. Il se compose de deux os, le talus ou l'astragale et le calcanéus ou calcanéum.

Le calcanéus constitue l'os le plus solide et volumineux du pied. La face supérieure du calcanéus s'articule avec le talus et sa face antérieure avec l'os cuboïde. Le calcanéus est composé du sustentaculum tali, projection osseuse située sur la face médiale et supérieure, permettant le soutien du talus, de la trochée fibulaire, petite crête se projetant sur la face latérale, de la tubérosité du calcanéus, constituant la face postérieure saillante et formant le talon.

La face médiale est composée d'une partie antérosupérieure et d'une partie inférieure. La partie antérosupérieure présente une volumineuse saillie appelée sustentaculum tali qui supporte à sa face supérieure, les facettes talaires antérieure et moyenne et à sa face inférieure le sillon permettant le passage des tendons des muscles longs fléchisseurs des orteils et de l'hallux. La partie inférieure de la face médiale quant à elle constitue le site d'insertion du muscle carré plantaire.

En contact direct avec le sol, le calcanéus supporte l'ensemble du poids du corps. Il redistribue les forces et les contraintes statiques et dynamiques vers les structures jambières et participe à la fonction de bras de levier. Il permet l'équilibre de l'arrière-pied et a un rôle d'amortisseur des impacts liés à la marche.

La station debout prolongée peut engendrer des lésions des différents éléments constitutifs du talon et provoquer des douleurs importantes.

Lorsque la douleur est située au niveau inférieur du talon, on peut la mettre en lien avec une fasciite plantaire c'est à dire l'inflammation des ligaments reliant les os des orteils au calcanéum au niveau de l'aponévrose plantaire. Lors de traumatismes violents ou dans des formes avancées, elles peuvent engendrer la formation d'une épine calcanéenne appelée aussi « épine de Lenoir » correspondant à une calcification de l'insertion du tendon des muscles longs fléchisseurs des orteils au niveau du calcanéus.

Les traitements recommandés de ces affections sont les étirements, l'application de glace, le changement de chaussures, la pose de strappings adhésifs ou de bandages soutenant la voûte plantaire, le port de talonnettes dans les chaussures destinées à amortir, soutenir et rehausser le talon, voir la prise d'anti-inflammatoires non stéroïdiens en première intention ou l'infiltration de corticoïdes.

Bien que les talonnettes soient réalisées en matériaux mous tel que du silicone, cette surépaisseur crée sous le talon, augmentent l'appui du talon et par conséquent l'inflammation.

La présente invention propose un dispositif orthopédique permettant d'exercer une pression au niveau de la partie postérieure du fascia plantaire, en inhibant l'appui prolongé du talon.

A cet effet, et selon un premier aspect, l'invention concerne un dispositif orthopédique destiné à être fixé à une semelle intérieure d'une chaussure, et caractérisé en ce qu'il comprend un moyen de décharge talonnière comprenant une extrémité supérieure destinée à exercer une pression à l'aplomb de la jonction de la partie postérieure de la voûte plantaire et de la partie antérieure du talon, et en ce que le moyen de décharge talonnière a une épaisseur maximale comprise entre 6 et 14 millimètres et en ce que sa dureté shore A soit comprise entre 15 et 40.

Selon une caractéristique, chacune des sections transversales à la direction longitudinale antéro-postérieure du moyen de décharge talonnière, a une épaisseur constante ou sensiblement constante sur toute sa largeur, à savoir selon une direction transversale interne-externe.

Selon des modes de réalisation, l'extrémité supérieure du moyen de décharge talonnière a une forme concave destinée à épouser la partie convexe de la partie antérieure du talon.

Selon une autre caractéristique, l'épaisseur maximale du moyen de décharge talonnière est comprise entre 8 et 12 millimètres

Selon des modes de réalisation, le moyen de décharge talonnière comprend une face inférieure destinée à être fixée à la semelle intérieure, une face supérieure inclinée et une face postérieure concave s'étendant de l'extrémité supérieure de la face supérieure à l'extrémité de la face inférieure agencée en correspondance, la face postérieure formant un angle aigu avec la face inférieure.

Selon une caractéristique supplémentaire, le dispositif orthopédique comprend un moyen de positionnement du moyen de décharge talonnière, s'étendant au-delà de l'extrémité postérieure du moyen de décharge talonnière et dont l'extrémité postérieure du moyen de positionnement est destinée à venir se positionner à l'extrémité postérieure de la semelle intérieure.

Selon la caractéristique précédente, le moyen de positionnement est une languette souple assemblée de manière non permanente à la partie supérieure du moyen de décharge talonnière.

Selon un mode de réalisation, le moyen de décharge talonnière est un moyen de décharge talonnière antérieur comprenant une face inférieure, une face supérieure inclinée, une face postérieure concave destinée à épouser la partie antérieure du talon, la jonction de la face supérieure et de la face postérieure formant une arête correspondant à l'extrémité supérieure du moyen de décharge talonnière antérieur.

Selon un second aspect, l'invention concerne une semelle orthopédique comprenant un portion antérieure, une surépaisseur et une portion postérieure, la portion antérieure étant destinée à supporter l'avant pied et la partie antérieure de la voûte plantaire, la portion postérieure étant destinée à l'appui du talon, la surépaisseur comprenant une extrémité supérieure destinée à exercer une pression à l'aplomb de la jonction de la partie postérieure de la voûte plantaire et de la partie antérieure du talon, caractérisée en ce que la surépaisseur a une épaisseur maximale comprise entre 6 et 14 millimètres et une dureté shore A soit comprise entre 15 et 40.

Selon une caractéristique, chacune des sections transversales à la direction longitudinale antéro-postérieure, de la surépaisseur, a une épaisseur constante ou sensiblement constante sur toute sa largeur, à savoir selon une direction transversale interne-externe.

La mise en oeuvre de l'invention sera mieux comprise à l'aide de la description détaillée qui est exposée ci-après en regard des dessins annexés dans lesquels :

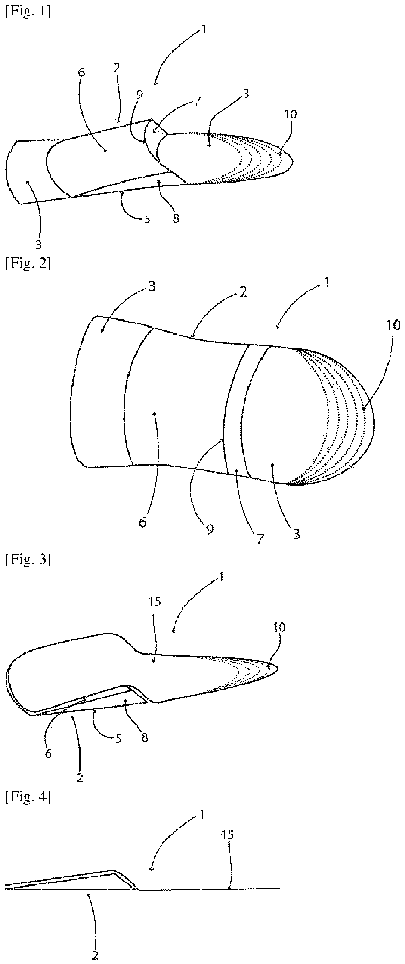

Les figures 1 et 2 illustrent un dispositif orthopédique comprenant un moyen de décharge talonnière, selon un mode de réalisation de l'invention.
[Fig 1] est une vue en perspective.
[Fig 2] est une vue de dessus de la figure 1.
Les figures 3 et 4 illustrent un dispositif orthopédique comprenant un moyen de décharge talonnière, selon un autre mode de réalisation.
[Fig 3] est une vue en perspective.
[Fig 4] est une vue en élévation de la figure 3.

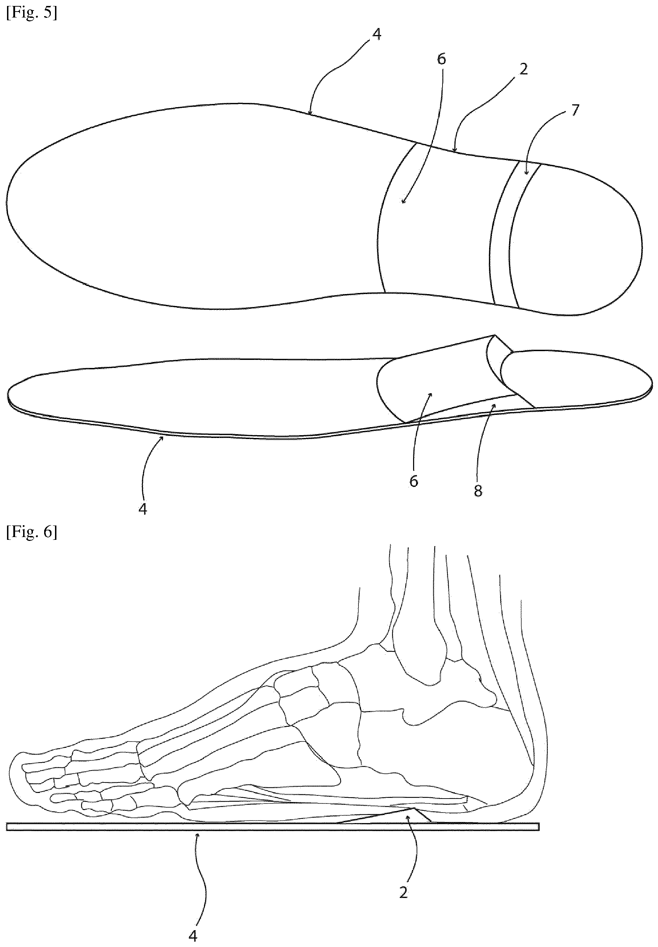

Les figures 5 et 6 illustrent un moyen de décharge talonnière tel qu'illustré à la figure 1 ou 3, fixé à une semelle intérieure de chaussure.
[Fig 5] illustre une vue de dessus d'une semelle intérieure droite et une vue en perspective d'une semelle intérieure gauche, sur chacune desquelles est fixé un moyen de décharge talonnière.
[Fig 6] est une vue en élévation schématique d'un pied gauche, dans lequel le système osseux et une partie des ligaments et tendons sont représentés, le pied reposant sur une semelle intérieure équipée d'un moyen de décharge talonnière tel qu'illustré à la figure 1.

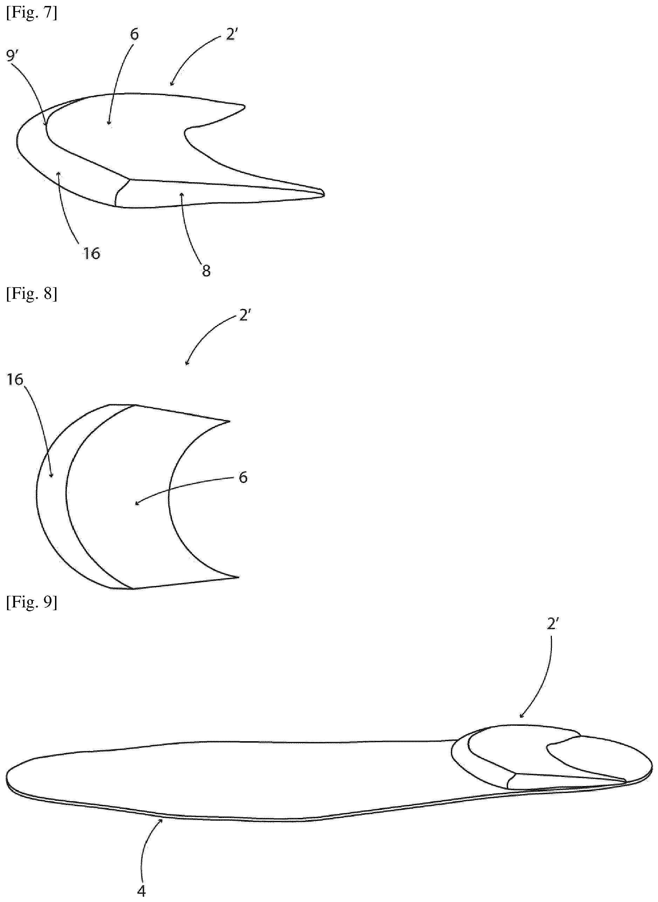

Les figures 7 à 10 illustrent un moyen de décharge talonnière d'un dispositif orthopédique selon un autre mode de réalisation.
[Fig 7] est une vue en perspective.
[Fig 8] est une vue de dessus de la figure 7.
[Fig 9] illustre une vue en perspective d'une semelle intérieure gauche sur laquelle est fixé un moyen de décharge talonnière.

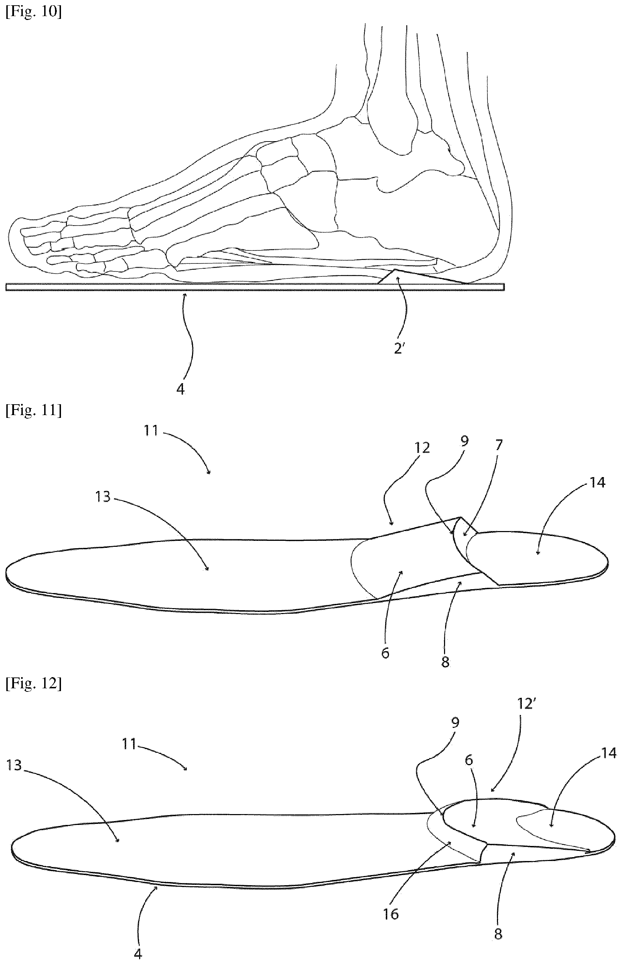

[Fig 10] est une vue en élévation schématique d'un pied gauche, dans lequel le système osseux et une partie des ligaments et tendons sont représentés, le pied reposant sur une semelle intérieure équipée d'un moyen de décharge talonnière tel qu'illustré à la figure 7.
[Fig 11] est une vue en perspective d'une semelle orthopédique selon un mode d'exécution.
[Fig 12] est une vue en perspective d'une semelle orthopédique selon un autre mode d'exécution.

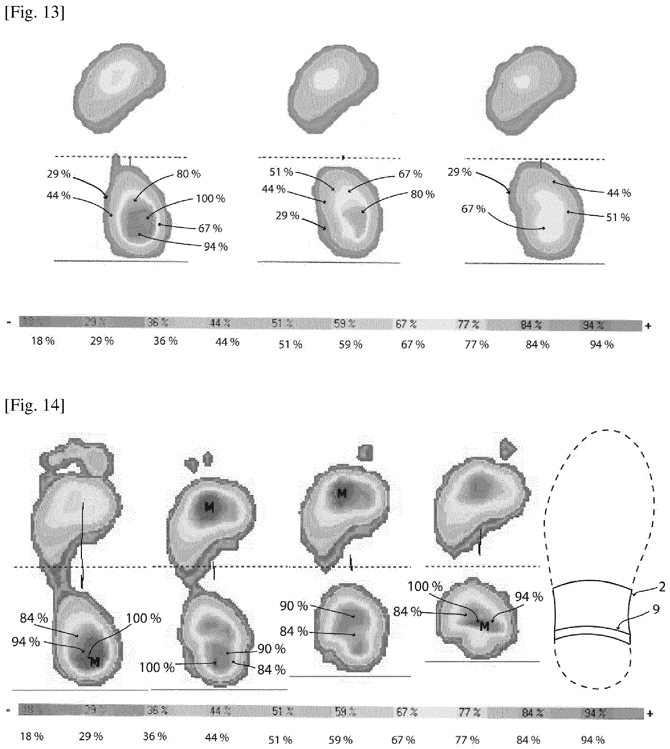

[Fig 13] illustre trois diagrammes de mesure de pression d'un pied gauche, respectivement de gauche à droite : sans dispositif orthopédique, en présence du moyen de décharge talonnière illustré à la figure 7 et du moyen de décharge talonnière illustré à la figure 1, les moyens de décharge talonnière ont une épaisseur maximale de 8 millimètres.
[Fig 14] illustre quatre diagrammes de mesure de pression d'un pied gauche et une vue du moyen de décharge talonnière illustré à la figure 1, respectivement de gauche à droite, sans dispositif orthopédique, en présence du moyen de décharge talonnière illustré à la figure 1 ayant une épaisseur maximale de 8 millimètres, en présence du moyen de décharge talonnière illustré à la figure 1 ayant une épaisseur maximale de 9 millimètres, en présence du moyen de décharge talonnière illustré à la figure 1 ayant une épaisseur maximale de 10 millimètres.

Les modes de réalisation illustrés aux figures 7 à 10 et 12 ne sont pas inclus dans l'objet des revendications.

Dans la suite de la description, il sera fait référence à une partie antérieure qui est placée avant, devant, en opposition à une partie postérieure qui est placée en arrière.

Il sera fait référence à une direction antéro-postérieure, une direction longitudinale correspondant à la direction de la ligne médiane d'une semelle intérieure, de l'avant vers l'arrière.

La voûte plantaire forme une arche composée d'une partie antérieure descendante en direction de l'extrémité antérieure de l'avant pied et d'une partie postérieure descendante en direction du talon.

Selon un premier aspect, le dispositif orthopédique (1) selon l'invention comprend un moyen de décharge talonnière (2), tel qu'illustré aux figures 1 à 4, , qui est destiné à limiter l'appui du talon et à exercer une pression sur la partie postérieure du fascia plantaire à la jonction de la partie antérieure du talon, en regard de la face médiale du calcanéum, tel qu'illustré à la figure 6.

Selon les modes de réalisation illustré, le dispositif orthopédique (1) comprend un moyen de décharge talonnière (2) et un moyen de positionnement du moyen de décharge talonnière (2) sur une semelle intérieure (4) d'une chaussure.

Le moyen de décharge talonnière (2) comprend une extrémités supérieure destinée à exercer une pression à la j onction de la voûte plantaire et de la partie antérieure du talon.

Le moyen de décharge talonnière (2) a un double effet technique cumulé, à savoir un effet mécanique par l'inhibition de l'appui du talon et un effet physiologique par l'atténuation de la tension de l'insertion des tendons des fléchisseurs au niveau du calcanéus.

Ces effets mécanique et physiologique détendent les muscles de la voûte plantaire et inhibe l'effet reflex du fascia plantaire lors de l'appui du pied.

La présence du moyen de décharge talonnière (2) crée également une détente du talon d'Achille.

Ainsi, le dispositif orthopédique (1) selon l'invention permet d'inhiber l'appui prolongé du talon dans une position statique et lors de la marche.

Le moyen de décharge talonnière (2) permet ainsi d'inhiber les douleurs liées à l'inflammation dû à la présence d'une épine calcanéenne, réduisant l'affection et limitant voir résolvant l'affection médicale résultante.

Selon les modes de réalisation précédent, on qualifiera le moyen de décharge talonnière (2) selon sa position relative à la partie antérieure du talon, à savoir un moyen de décharge talonnière antérieur (2) tel qu'illustré à la figure 1.

Pour la compréhension de l'invention, les éléments communs au moyen de décharge talonnière antérieur (2) et au moyen de décharge talonnière postérieur (2) portent les mêmes références.

Selon les modes de réalisation illustrés, le moyen de décharge talonnière (2) se présente par une pièce avantageusement monobloc moulée par thermoformage ou façonnée au dimensionnement requis tels que décrit plus en détail dans la suite de la description.

Selon une caractéristique, chacune des sections transversales à la direction longitudinale antéro-postérieure du moyen de décharge talonnière (2), à savoir du moyen de décharge talonnière antérieur (2), ont une épaisseur constante ou sensiblement constante sur toute sa largeur, à savoir selon une direction transversale interne-externe.

Selon les modes réalisation illustrés aux figures 1 à 6, le moyen de décharge talonnière antérieur (2) comprend une partie postérieure destinée à être positionnée en regard de la face médiale du calcanéus, et une partie antérieure destinée à venir supporter la partie postérieure de la voûte plantaire. La partie postérieure et la partie antérieure du moyen de décharge talonnière (2) sont destinées à être orientées selon la direction antéro-postérieure d'une semelle antérieure (4) d'une chaussure.

Le moyen de décharge talonnière antérieur (2) comprend une face inférieure (5) destinée à être fixée à la semelle intérieure (4), une face supérieure (6) d'appui inclinée destinée à supporter la partie postérieure de la voûte plantaire et une face postérieure (7) s'étendant de l'extrémité postérieure de la face inférieure (5) à l'extrémité supérieure de la face supérieure (6) d'appui qui est orientée en direction postérieure, la face postérieure (7) formant un angle aigu avec la face inférieure (5).

Le moyen de décharge talonnière antérieur (2) comprend également deux faces latérales (8) délimitées par les extrémités latérales intérieures et extérieures respectives de la face inférieure (5), de la face supérieure (6) et de la face postérieure (7).

Les faces latérales (8) sont destinées à venir au contact des parois latérales de la tige de la chaussure.

La face postérieure (7) est destinée à venir au contact de la partie antérieure du talon, plus précisément en regard de la partie antérieure du calcanéus.

La jonction de la face supérieure (6) et de la face postérieure (7) forme une arête (9) correspondant à l'extrémité supérieure du moyen de décharge talonnière antérieur (2).

Rappelons que la face postérieure (7) est destinée à venir au contact de la partie antérieure du talon à la jonction de la partie postérieure de la voûte plantaire.

A cet effet, selon une caractéristique supplémentaire, la face postérieure (7) est concave, afin d'épouser la forme convexe de la partie antérieure du talon.

L'arête (9) du moyen de décharge talonnière antérieur (2) a ainsi une forme concave épousant la forme convexe de la jonction de la voûte planaire et de la partie antérieure du talon.

Chacune des sections transversales à la direction longitudinale antéro-postérieure du moyen de décharge talonnière antérieur (2) ayant une épaisseur constante ou sensiblement constante sur toute sa largeur, la pression exercée par le moyen de décharge talonnière antérieur (2) est ainsi répartie uniformément sur la largeur de la partie postérieure de la voûte plantaire et surtout à la jonction de la partie postérieure de la voûte plantaire et la partie antérieure d'extrémité du talon.

La forme concave de la face postérieure (7) du moyen de décharge talonnière antérieur (2), cumulée à l'épaisseur constante ou sensiblement constante sur sa largeur, permet à l'arête (9) formée par la j onction de la face supérieure (6) et de la face postérieure (7), d'exercer une pression uniformément répartie au niveau de l'insertion des tendons des fléchisseurs sur le calcanéus, sur toute la largeur de la jonction de la partie postérieure de la voûte plantaire et de la partie antérieure d'extrémité du talon.

Précisons que la jonction de la face inférieure (5) et de la face postérieure (7) est destinée à venir être compressée par la base antérieure du talon, à l'extrémité postérieure de la voûte plantaire.

L'arête (9) formée par la j onction de la face postérieure (7) et de la face supérieure (6) est la localisation de pression maximale exercée sur la partie postérieure du fascia plantaire.

Selon le mode de réalisation illustré aux figures 7 à 9, le moyen de décharge talonnière postérieur (2') comprend une partie postérieure destinée à venir supporter la partie antérieure du talon, et une partie antérieure destinée à être positionner sensiblement à l'aplomb de de la jonction de la voûte plantaire et du talon.

La base du talon, correspondant à la localisation d'appui maximale du talon, est destinée à reposer sur la semelle intérieure de la chaussure.

Les figures 7 et 8 n'illustrent pas de moyen de positionnement du moyen de décharge talonnière postérieur (2') sur une semelle intérieure d'une chaussure.

Les moyens de positionnement illustrés aux figures 1 et 3 sont parfaitement adapté et intégrable aux moyens de décharge talonnière postérieur (2') illustré, tel que décrit plus en détail dans la suite de la description.

Le moyen de décharge talonnière postérieur (2') comprend une face inférieure (5) destinée à être fixée à la semelle intérieure (4), une face supérieure (6) d'appui inclinée destinée à supporter la partie antérieure du talon et une face antérieure (16) s'étendant de l'extrémité antérieure de la face inférieure (5) à l'extrémité supérieure de la face supérieure (6) d'appui, la face antérieure (16) formant un angle aigu avec la face inférieure (5).

La jonction de la face supérieure (6) et de la face antérieure (16) forme une arête (9).

L'extrémité supérieure de la face supérieure (6) inclinée est orientée en direction antérieure.

L'extrémité inférieure de la face supérieure (6) est destinée à rejoindre la surface supérieure de la partie postérieure de la semelle intérieure (4).

Précisons que la jonction de la face inférieure (5) et de la face supérieure (6) est destinée à venir être compressée par la base postérieure du talon.

La face antérieure (16) est destinée à venir au contact de l'extrémité de la partie postérieure de la voûte plantaire lors de l'appui du pied dans la chaussure.

Le moyen de décharge talonnière postérieur (2') comprend également deux faces latérales (8) délimitées par les extrémités latérales intérieures et extérieures respectives de la face inférieure (5), de la face supérieure (6) et de la face antérieure (16).

Rappelons que la face antérieure (16) est destinée à se positionner à l'aplomb de la partie antérieure du talon à la jonction de la partie postérieure de la voûte plantaire.

A cet effet, selon une caractéristique supplémentaire, la face antérieure (16) est convexe, afin de correspondre à la forme convexe de la partie antérieure du talon.

Ainsi, l'arête (9) a une forme convexe destinée à épouser la forme convexe de la partie antérieure du talon.

Chacune des sections transversales à la direction longitudinale antéro-postérieure du moyen de décharge talonnière postérieur (2') ayant une épaisseur constante ou sensiblement constante sur toute sa largeur, la pression exercée par le moyen de décharge talonnière antérieur (2) est ainsi répartie uniformément sur la largeur de la partie postérieure de la voûte plantaire et surtout à la jonction de la partie postérieure de la voûte plantaire et la partie antérieure d'extrémité du talon.

La forme convexe de la face antérieure (16) du moyen de décharge talonnière postérieur (2'), cumulée à l'épaisseur constante ou sensiblement constante sur sa largeur, permet à l'arête (9) formée par la jonction de la face supérieure (6) et de la face antérieure (16), d'exercer une pression uniformément répartie au niveau de l'insertion des tendons des fléchisseurs sur le calcanéus, sur toute la largeur de la jonction de la partie postérieure de la voûte plantaire et de la partie antérieure d'extrémité du talon.

L'arête (9) formée par la jonction de la face postérieure (7) et de la face supérieure (6) est la localisation de pression maximale exercée sur la partie postérieure du fascia plantaire.

La pression sur le fascia plantaire, inhibant l'appui excessif du talon, induisant les effets mécaniques et physiologiques précités, est réalisée par une épaisseur et une dureté spécifique du moyen de décharge talonnière (2).

Selon une caractéristique, le moyen de décharge talonnière (2) a une épaisseur maximale à son extrémité supérieure comprise entre 6 et 14 millimètres, de préférence comprise entre 7 et 13 millimètres, plus préférentiellement comprise entre 8 et 12 millimètres.

Plus précisément, selon la caractéristique précédente, l'épaisseur maximale de l'extrémité supérieure du moyen de décharge talonnière (2) est localisée au niveau de l'arête (9), qui est comprise entre 6 et 14 millimètres, de préférence comprise entre 7 et 13 millimètres, plus préférentiellement comprise entre 8 et 12 millimètres.

Rappelons que tel qu'illustré à la figure 1, la face inférieure (5) et la face postérieure (7) du moyen de décharge talonnière antérieur (2), forme un angle aigu, la face postérieure (7) étant donc orientée en direction de la partie antérieure du dispositif orthopédique (1), à savoir destinée à être orientée en direction de l'avant pied.

Rappelons que tel qu'illustré à la figure 7, la face inférieure (5) et la face antérieure (7) du moyen de décharge talonnière postérieur (2'), forme un angle obtu, la face antérieure (16) étant donc orientée en direction de la partie postérieure du dispositif orthopédique (1), à savoir destinée à être orientée en direction du talon.

Selon le mode de réalisation illustré à la figure 1, selon une projection perpendiculaire à la face inférieure (5), selon une vue de dessus, la distance de l'extrémité postérieure de la face inférieure (5) à l'extrémité supérieure de la face supérieure (6) d'appui du moyen de décharge talonnière antérieur (2), bordant la face postérieure (7), est comprise entre 7 et 10 millimètres.

Selon le mode de réalisation illustré à la figure 7, selon une projection perpendiculaire à la face inférieure (5), selon une vue de dessus, la distance de l'extrémité antérieure de la face inférieure (5) à l'extrémité supérieure de la face supérieure (6) d'appui du moyen de décharge talonnière postérieur (2'), bordant la face antérieure (16), est comprise entre 7 et 10 millimètres.

La largeur du moyen de décharge talonnière (2) est comprise entre 50 et 60 millimètres.

La longueur du moyen de décharge talonnière (2) est comprise entre 50 et 70 millimètres, de préférence comprise entre 55 et 65 millimètres.

Selon une autre caractéristique, la dureté shore A du matériau constitutif du moyen de décharge talonnière (2) est comprise entre 15 et 40.

De façon connue de l'homme du métier, la dureté est mesurée à température ambiante à l'aide d'un Duromètre normalisé. Le principe de mesure de la dureté shore d'un matériau consiste en le calcul de la résistance du matériau mesuré à la pénétration d'une sonde avec une charge prédéfinie.

Une épaisseur maximale et une dureté relativement importante sont nécessaires pour assurer une pression suffisante au niveau de la partie postérieure du fascia plantaire en regard du calcanéus, afin d'inhiber l'appui du talon et d'atténuer la tension de l'insertion des tendons des fléchisseurs, sans que le matériau constitutif du moyen de décharge talonnière (2) soit trop compressé par le poids de l'utilisateur de telle manière à ce que ses effets soient rendus inopérants.

L'épaisseur maximale et la dureté du moyen de décharge talonnière (2) sont modulées dans les gammes précitées selon le poids de l'utilisateur.

A titre d'exemple non limitatif, le matériau constitutif du moyen de décharge talonnière (2) peut être choisis selon la dureté souhaitée, parmi du latex naturel, du polyéthylène réticulé, du polyéthylène acétate de vinyle réticulé ou d'un mélange de polyéthylène et de polyéthylène acétate de vinyle réticulés.

La dureté des mousses de polyoléfines précitées peut être modulée par le taux de réticulation, par exemples par la modularité de la quantité de peroxydes ou de silanes introduits dans le mélange de granulés de polymères.

Une large gamme des matériaux précités sont disponibles commercialement.

Selon un premier exemple de réalisation, le matériau constitutif du moyen de décharge talonnière (2) est constitué de mousse de latex naturel, ayant une densité de 320 kg/m³, une dureté shore A de 17 et une épaisseur maximale de 8 millimètres.

Selon un deuxième exemple de réalisation, le matériau constitutif du moyen de décharge talonnière (2) est constitué de mousse de latex naturel, ayant une densité de 320 kg/m³, une dureté shore A de 17 et une épaisseur maximale de 10 millimètres.

Selon un troisième exemple de réalisation, le matériau constitutif du moyen de décharge talonnière (2) est constitué de mousse de latex naturel, ayant une densité de 320 kg/m³, une dureté shore A de 17 et une épaisseur maximale de 14 millimètres.

Selon un quatrième exemple de réalisation, le matériau constitutif du moyen de décharge talonnière (2) est constitué de mousse de latex naturel, ayant une densité de 400 kg/m³, une dureté shore A de 21 et une épaisseur maximale de 10 millimètres.

Selon un cinquième exemple de réalisation, le matériau constitutif du moyen de décharge talonnière (2) est constitué d'une mousse de polyéthylène réticulé, ayant une densité de 140 kg/m³, une dureté shore A de 26 et une épaisseur maximale de 12 millimètres.

Selon un sixième exemple de réalisation, le matériau constitutif du moyen de décharge talonnière (2) est constitué d'une mousse de polyéthylène réticulé, ayant une densité de 160 kg/m³, une dureté shore A de 35 et une épaisseur maximale de 6 millimètres.

Selon un septième exemple de réalisation, le matériau constitutif du moyen de décharge talonnière (2) est constitué d'une mousse de polyéthylène acétate de vinyle réticulé, ayant une densité de 200 kg/m³, une dureté shore A de 40 et une épaisseur maximale de 8 millimètres.

Selon un huitième exemple de réalisation, le matériau constitutif du moyen de décharge talonnière (2) est constitué d'une mousse de polyéthylène acétate de vinyle réticulé, ayant une densité de 200 kg/m³, une dureté shore A de 40 et une épaisseur maximale de 12 millimètres.

Selon un neuvième exemple de réalisation, le matériau constitutif du moyen de décharge talonnière (2) est constitué d'une mousse formée d'un mélange de polyéthylène et de polyéthylène acétate de vinyle réticulés, ayant une densité de 120 kg/m³, une dureté shore A de 20 et une épaisseur maximale de 8 millimètres.

Différentes gammes de dispositifs orthopédiques (1) selon l'invention pourront être disponibles pour les utilisateurs, fournies avec une notice d'utilisation indiquant quel type de dispositif orthopédique (1), à savoir le type de matière et l'épaisseur, est recommandé selon la morphologie de l'utilisateur.

Un mauvais positionnement du dispositif orthopédique (1) par rapport à la semelle intérieure (4) peut induire un basculement du bassin et induire une posture inadéquate.

Le dispositif orthopédique (1) selon les modes de réalisation précédents, comprend un moyen de positionnement du moyen de décharge talonnière (2), de telle manière à ce que l'extrémité postérieure du moyen de positionnement lors de sa mise en place sur une semelle intérieure (4), vienne se positionner à l'extrémité postérieure de la semelle intérieure (4), en butée contre la tige de la chaussure à l'extrémité postérieure du talon, afin de positionner notamment l'extrémité supérieure du moyen de décharge talonnière (2), à savoir l'arête (9) de manière optimale.

Selon les modes de réalisation illustrés au figures 1 à 4, le moyen de positionnement est soit un film adhésif (3) tel qu'illustré à la figure 1 soit une languette (15) telle qu'illustrée à la figure 3.

Selon le mode de réalisation illustré à la figure 1, le dispositif orthopédique (1) comprend un film adhésif (3) solidaire de la face inférieure du moyen de décharge talonnière antérieur (2), dont la face inférieure du film adhésif (3) est destinée à être fixée solidairement à une semelle intérieure (4) d'une chaussure.

Le film adhésif (3) comprend une partie centrale sur laquelle le moyen de décharge talonnière (2) est solidarisé, une partie postérieure correspondante à la zone d'appui du talon, dont son extrémité postérieure est destinée à venir se positionner à l'extrémité postérieure d'une semelle intérieure (4) contre la partie de la tige de la chaussure entourant le talon, et avantageusement une partie antérieure destinée à améliorer la tenue du film adhésif (3) sur la semelle intérieure (4) par l'augmentation de la surface de collage.

Plus précisément, la face inférieure (5) du moyen de décharge talonnière (2) est solidarisé à la surface supérieure de la partie centrale du film adhésif (3).

Selon le mode d'exécution, illustré aux figures 1 et 2, le film adhésif (3) comprend une partie postérieure, une partie centrale et une partie antérieure.

Etant donné la faible épaisseur du film adhésif (3), ce dernier n'est pas illustré sur les figures 5, 6, 9 et 10.

Selon un autre mode d'exécution, non illustré, le film adhésif (3) est constitué d'une partie centrale et d'une partie postérieure.

Plus précisément, tel qu'illustré aux figures 1 et 2, la partie supérieure du film adhésif (3) s'étend sous la face inférieure du moyen de décharge talonnière antérieur (2) et se prolonge en direction postérieure au-delà de la jonction de la face inférieure (5) et de la face postérieure (7), ainsi qu'avantageusement en direction antérieure, au-delà de la jonction de la face inférieure (5) et de la face supérieure (6).

Selon un autre mode de réalisation non illustré, le dispositif orthopédique (1) comprend un moyen de décharge talonnière postérieur (2') et un film adhésif (3) tel que décrit précédemment.

Selon le mode de réalisation précédent, le film adhésif (3) s'étend sous la face inférieure du moyen de décharge talonnière postérieur (2') et se prolonge en direction postérieure au-delà de la jonction de la face inférieure (5) et de la face supérieure (6).

La partie postérieure du film adhésif (3) comprend des lignes de démarcation (10) correspondant aux différentes pointures, la partie postérieure d'extrémité excédante du film adhésif (3) étant destinée à être désolidarisée, par exemple par découpage, pour s'adapter à la pointure de l'utilisateur.

Les lignes de démarcation (10) sont courbes destinées à épouser la forme convexe de l'extrémité postérieure de la semelle intérieure (4) d'une chaussure, et sont séparées les unes des autres d'une distance correspondant à une pointure, qui est environ égale à 5 millimètres. Les lignes de démarcation (10) sont par exemple présentes en un nombre égal au pointures couramment utilisées, par exemple correspondantes aux pointures européennes du 36 au 48 ou américaines de 5 à 13, ou britannique de 3,5 à 12,5.

Une fois la partie excédentaire du film adhésif (3) désolidarisée et la partie restante du film adhésif (3) fixée à une semelle intérieure (4), le dispositif orthopédique (1) est positionné de manière optimale pour que l'arête (9) du moyen de décharge talonnière (2) puisse exercer une pression à la jonction de la partie postérieure de la voûte plantaire et la partie antérieure du talon.

Selon le mode de réalisation illustré aux figures 3 et 4, le dispositif orthopédique (1) comprend une languette (15) en tant que moyen de positionnement du moyen de décharge talonnière (2).

La languette (15) est une bande en matériau synthétique souple assemblée de manière non permanente à la face supérieure (6) du moyen de décharge talonnière (2), et destinée à être désolidarisée du moyen de décharge talonnière (2) une fois ce dernier positionné et fixé à une semelle intérieure (4), tel qu'illustré aux figures 5, 6, 9 et 10.

L'assemblage non permanent de la languette (15) au moyen de décharge talonnière (2) est par exemple réalisée à l'aide du résine tackifiante.

A cet effet, selon le mode de réalisation précédent, le moyen de décharge talonnière (2) assemblé de manière non permanente à une languette (15), comprend un adhésif localisé sur sa surface inférieure (5). La surface inférieure (5) est équipée d'un opercule, non représenté, destiné à être retiré préalablement au positionnement du dispositif orthopédique (1) sur une semelle intérieure (4).

Tel qu'illustré aux figures 3 et 4, la languette (15) s'étend sur la face supérieure (6) du moyen de décharge talonnière antérieur (2) et se prolonge au-delà de la jonction de l'arête (9).

Selon un autre mode de réalisation non illustré, la languette (15) s'étend sur la face supérieure du moyen de décharge talonnière postérieur (2') et se prolonge au-delà de la jonction de la face supérieure (6) et de la face inférieure (5).

Selon une caractéristique supplémentaire, la languette (15) comprend des lignes de démarcation (10) correspondant aux différentes pointures, la partie postérieure d'extrémité excédante de la languette (15) étant destinée à être désolidarisée, par exemple par découpage, pour s'adapter à la pointure de l'utilisateur.

Les lignes de démarcation (10) de la languette (15) ont des caractéristiques identiques à celles du film adhésif (3) décrit précédemment.

La mise en place du dispositif orthopédique (1) selon le mode de réalisation précédent, consiste en la désolidarisation, par exemple par découpage le long d'une ligne de démarcation (10), de la partie excédentaire de la languette (15) pour correspondre à la pointure de l'utilisateur, le retrait de l'opercule, la mise en place de l'extrémité postérieure de la languette (15) en correspondance de l'extrémité postérieure de la semelle intérieure (4), le déroulée de la languette (15) contre la surface supérieure de la semelle intérieure (4), puis du déroulé du moyen de décharge talonnière (2) qui vient se coller sur la semelle intérieure (4), suivit du retrait de la languette (15) du moyen de décharge talonnière (2).

Selon un second aspect, l'invention concerne une semelle orthopédique (11) intégrant une surépaisseur (12), conforme au moyen de décharge talonnière (2) du dispositif orthopédique (1) selon l'invention, introduit précédemment.

La semelle orthopédique (11) est destinée à être introduite dans une chaussure, en remplacement de la semelle intérieure de la chaussure, si la semelle intérieure est amovible, ou introduite au-dessus de la semelle intérieure de la chaussure, si la semelle intérieure est solidaire de la chaussure.

Les effets mécanique et physiologique de la surépaisseur (12) sont identiques et conformes à ceux du moyen de décharge talonnière (2) décrits précédemment.

Les gammes de dimensionnement et de dureté shore A, ainsi que les formes de la surépaisseur (12) sont identiques à celles du moyen de décharge talonnière (2) décrites précédemment.

Ainsi, la surépaisseur (12) a une épaisseur maximale comprise entre 6 et 14 millimètres, de préférence comprise entre 7 et 13 millimètres, plus préférentiellement comprise entre 8 et 12 millimètres et une dureté shore A soit comprise entre 15 et 40, selon la morphologie de l'utilisateur.

Il est entendu que l'épaisseur maximale précité est mesurée de la base de la face postérieure (7) ou de la base de la face antérieure (16) à l'extrémité supérieure de la face supérieure (6), à savoir de l'arête (9).

La surépaisseur (12) faisant partie intégrante de la semelle orthopédique (11), les différences entre la surépaisseur (12) et le dispositif orthopédique (1) sont l'absence du moyen de positionnement et de la surface inférieure (5) du moyen de décharge talonnière (2).

A cet effet, selon le mode de réalisation illustré à la figure 11, la semelle orthopédique (11) comprend une portion antérieure (13) destinée à supporter l'avant pied et la partie antérieure de la voûte plantaire, une surépaisseur (12) destinée à supporter la partie postérieure ou l'extrémité de la partie postérieure de la voûte plantaire et une portion postérieure (14) d'appui du talon.

La portion antérieure (13) et la portion postérieure (14) sont avantageusement planes ou sensiblement planes, tout du moins ayant une épaisseur inférieure à celle de la surépaisseur (12).

La surépaisseur (12) comprend une extrémité supérieure destinée à être localisée à la jonction de la voûte plantaire et de la partie antérieure du talon.

Selon le mode de réalisation illustré à la figure 11, la surépaisseur antérieure (12) est formée d'un plan incliné, en tant que face supérieure (6) d'appui de la partie postérieure de la voûte plantaire, dont son extrémité supérieure est prolongée par une face postérieure (7) faisant un angle obtu avec la surface supérieure de la portion postérieure (14) de la semelle orthopédique (11).

Selon le mode de réalisation précédent, l'arête (9) formée par la jonction de la face supérieure (6) et de la face postérieure (7) de la surépaisseur antérieure (12), est destinée à exercer une pression à la jonction de la voûte plantaire et de la partie antérieure du talon, au niveau de l'insertion des tendons des fléchisseurs au niveau du calcanéus.

La surépaisseur antérieure (12) comprend ainsi une face supérieure (6) formant le plan incliné précité, une face postérieure (7) et deux faces latérales (8).

La face postérieure (7) est concave destinée à épouser la forme convexe de la partie antérieure du talon.

La distance de la base de la face postérieure (7) à l'extrémité postérieure de la semelle orthopédique (11) est comprise entre 50 et 80 millimètres, selon la pointure de l'utilisateur.

Selon le mode de réalisation illustré à la figure 12, la surépaisseur postérieure (12') est formée d'un plan incliné, en tant que face supérieure (6) d'appui de la base de la partie antérieure du talon, dont son extrémité supérieure est prolongée par une face antérieure (16) faisant un angle obtu avec la surface supérieure de la portion antérieure (13) de la semelle orthopédique (11).

Selon le mode de réalisation précédent, l'arête (9) formée par la jonction de la face supérieure (6) et de la face antérieure (16) de la surépaisseur postérieure (12'), est destinée à exercer une pression à la jonction de la voûte plantaire et de la partie antérieure du talon, au niveau de l'insertion des tendons des fléchisseurs au niveau du calcanéus.

La surépaisseur postérieure (12') comprend ainsi une face supérieure (6) formant le plan incliné précité, une face antérieure (16) et deux faces latérales (8).

La face antérieure (16) est convexe destinée à correspondre à la forme convexe de la partie antérieure du talon.

La surépaisseur (12,) peut être constituée du même matériau constitutif des autres portions (13, 14) de la semelle orthopédique (11), cette dernière peut être moulée d'un seul tenant selon la forme et le dimensionnement conforme à l'invention.

La surépaisseur (12) peut être constituée d'un matériau différent de celui des autres portions (13, 14) constitutives de la semelle orthopédique (11), la surépaisseur (12) est assemblée lors de la fabrication de la semelle orthopédique (11), par exemple par collage avec un adhésif ou par collage thermique. Dans ce cas, la dureté de la surépaisseur (12) est avantageusement supérieure à celles des autres portions (13, 14) constitutives de la semelle orthopédique (11).

Concernant ce second aspect de l'invention, une gamme de semelles orthopédique (11) de pointures différentes est produite, la surépaisseur (12) étant positionnée ou moulée à la localisation optimale pour que l'arête (9) soit destinée à être positionnée à la jonction de la voûte plantaire et de la partie antérieure du talon.

Les diagrammes illustrés aux figures 13 et 14 ont été réalisées à l'aide d'une plateforme de podométrie commerciale, pilotée par un logiciel d'enregistrement d'acquisitions statiques, dynamiques et posturologiques, permettant l'édition de compte rendu avec des empreintes illustrant une répartition des pressions et des surfaces d'appuis. Les acquisitions des diagrammes précités ont été réalisées en mesures statiques. La plateforme de podométrie a une surface active de 150 par 50 centimètres et comprend 12 288 capteurs résistifs. Un capteur résistif a un dimensionnement de 8 millimètres carrés.

Les diagrammes illustrés aux figures 13 et 14 ont été produites avec un patient mesurant 180 centimètres, pesant 95 kilogrammes et ayant une pointure 42.

Les diagrammes illustrent les mesures de pression de l'ensemble d'un pied et se décompose en un ensemble de mesures antérieures représentant l'appui de l'avant pied et de mesures postérieures représentant l'appui du talon contre la plateforme de podométrie.

Les mesures de pression sont exprimées en pourcentages calculés à partir de la pression maximale mesurée par la plateforme podologique. La pression maximale mesurée, soit 100 pourcents, sur les diagrammes illustrés est d'environ 750 grammes par centimètres carrés. La dispersion de pression maximale entre les différents diagrammes illustrés est d'environ 50 grammes par centimètres carrés, soit environ 7%.

La figure 13 illustre de gauche à droite, un diagramme de référence réalisé sans dispositif orthopédique (1), un diagramme réalisé en présence d'un moyen de décharge talonnière postérieur (2') et un diagramme réalisé en présence d'un moyen de décharge talonnière antérieur (2), ayant chacun une épaisseur maximale de 8 millimètres au niveau de l'arête (9).

Le matériau constitutif du moyen de décharge talonnière antérieur (2) et du moyen de décharge talonnière postérieur (2') utilisé pour ces analyses, est une mousse de latex naturel ayant une dureté Shore A de 17 et une densité de 320 kg/m³.

Sur le diagramme de référence, la zone centrale des mesures postérieures correspondant à la zone centrale de l'appui du talon, sont sensiblement comprises entre 80 et 100 pourcents de la pression exercée, tandis que sur le diagramme réalisé en présence d'un moyen de décharge talonnière antérieur (2) ayant une épaisseur de 8 millimètres, les pressions exercées au niveau de la zone centrale de l'appui du talon avoisines les 70 % de la pression maximale (mesurée à 67%). Une réduction de pression d'environ 30 pourcents est donc obtenue au niveau du talon en présence d'un moyen de décharge talonnière antérieur (2) ayant une épaisseur maximale de 8 millimètres, pour un patient pesant 95 kilogrammes.

De manière équivalente, en comparaison avec le diagramme de référence, le diagramme réalisé en présence d'un moyen de décharge talonnière postérieur (2') ayant une épaisseur de 8 millimètres, tel qu'illustré à la figure 7, les pressions exercées au niveau de la zone centrale de l'appui du talon avoisines les 80 % de la pression maximale. Une réduction de pression d'environ 15 à 20 pourcents est donc obtenue au niveau de la zone d'appui du talon.

La figure 14 illustre de gauche à droite, un diagramme de référence réalisé sans dispositif orthopédique (1), et des diagrammes réalisés en présence d'un moyen de décharge talonnière antérieur (2) tel qu'illustré à la figure 1, ayant une épaisseur maximale respective de 8, 9 et 10 millimètres au niveau de l'arête (9), ainsi qu'un schéma de positionnement du moyen de décharge talonnière antérieur (2).

Tel que précisé précédemment par l'analyse comparative décrite à la figure 13, la présence d'un moyen de décharge talonnière antérieur (2) d'une épaisseur maximale de 8 millimètres réduit la pression exercée au niveau de la zone centrale l'appui du talon. L'augmentation de l'épaisseur maximale du moyen de décharge talonnière (2) à des valeurs de 9 et 10 millimètres tend à réduire fortement voire à supprimer la pression exercée au niveau de l'appui du talon.

Le troisième et le quatrième diagramme représentant les mesures de pression en présence d'un moyen de décharge talonnière antérieur (2) d'une épaisseur respective de 9 et 10 millimètres, illustrent une suppression totale de l'appui du talon à l'extrémité postérieure du talon en position statique, en recentrant l'appui du pied au niveau de la zone correspondant au moyen de décharge de talonnière (2).

On peut également constater sur le troisième et le quatrième diagramme, les mesures de pression révélant la forme du moyen de décharge talonnière antérieur (2).

On peut également observer sur le quatrième diagramme une pression maximale comprise entre 94 et 100 pourcents au niveau de l'arête (9).

En comparaison avec le diagramme de référence, le quatrième diagramme révèle ainsi un déplacement de la pression maximale exercée de la zone centrale d'appui du talon vers la zone correspondante à l'arête (9) du moyen de décharge talonnière antérieur (2).

## Revendications

1. Dispositif orthopédique (1) destiné à être fixé à une semelle intérieure ( 4) d'une chaussure, comprenant un moyen de décharge talonnière (2) qui comprend une extrémité supérieure destinée à exercer une pression à l'aplomb de la jonction de la partie postérieure de la voûte plantaire et de la partie antérieure du talon, le moyen de décharge talonnière (2) ayant » une épaisseur maximale comprise entre 6 et 14 millimètres, sa dureté shore A étant comprise entre 15 et 40, la dureté étant mesurée à température ambiante à l'aide d'un Duromètre shore A normalisé, le moyen de décharge talonnière (2) comprenant une face inférieure (5) destinée à être fixée à la semelle intérieure (4), une face supérieure (6) inclinée et une face postérieure (7) concave s'étendant de l'extrémité supérieure de la face supérieure (6) à l'extrémité de la face inférieure (5) agencée en correspondance, la face postérieure (7) formant un angle aigu avec la face inférieure (5), la jonction de la face supérieure (6) inclinée et de la face postérieure (7) formant une arête (9) correspondant à l'extrémité supérieure du moyen de décharge talonnière (2), l'arête (9) étant destinée à exercer une pression au niveau de l'insertion des tendons des fléchisseurs au niveau du calcanéus.

2. Dispositif orthopédique (1) selon la revendication 1, **caractérisé en ce que** chacune des sections transversales à la direction longitudinale antéro-postérieure du moyen de décharge talonnière (2), a une épaisseur constante ou sensiblement constante sur toute sa largeur, à savoir selon une direction transversale interne-externe

3. Dispositif orthopédique (1) selon la revendication 1 ou 2, **caractérisé en ce que** l'extrémité supérieure du moyen de décharge talonnière (2) a une forme concave destinée à épouser la partie convexe de la partie antérieure du talon.

4. Dispositif orthopédique (1) selon l'une quelconque des revendications précédentes, **caractérisé en ce que** l'épaisseur maximale du moyen de décharge talonnière (2) est comprise entre 8 et 12 millimètres

5. Dispositif orthopédique (1) selon l'une quelconque des revendications précédentes, **caractérisé en ce qu'**il comprend un moyen de positionnement du moyen de décharge talonnière (2), s'étendant au-delà de l'extrémité postérieure du moyen de décharge talonnière (2) et dont l'extrémité postérieure du moyen de positionnement est destinée à venir se positionner à l'extrémité postérieure de la semelle intérieure (4).

6. Dispositif orthopédique (1) selon la revendication précédente, **caractérisé en ce que** le moyen de positionnement est une languette (15) souple assemblée de manière non permanente à la partie supérieure du moyen de décharge talonnière (2).

7. Semelle orthopédique (11) comprenant un portion antérieure (13), une surépaisseur (12) et une portion postérieure (14), la portion antérieure (13) étant destinée à supporter l'avant pied et la partie antérieure de la voûte plantaire, la portion postérieure (14) étant destinée à l'appui du talon, la surépaisseur (12) comprenant une extrémité supérieure destinée à exercer une pression à l'aplomb de la jonction de la partie postérieure de la voûte plantaire et de la partie antérieure du talon, la surépaisseur (12) a une épaisseur maximale comprise entre 6 et 14 millimètres et une dureté shore A comprise entre 15 et 40, la dureté est mesurée à température ambiante à l'aide d'un Duromètre shore A normalisé, la surépaisseur (12) comprenant une face supérieure (6) inclinée et une face postérieure (7) concave s'étendant de l'extrémité supérieure de la face supérieure (6) à la portion postérieure (14) ou la portion antérieure (13) respectivement, la jonction de la face supérieure (6) inclinée et de la face postérieure (7) formant une arête (9) correspondant à l'extrémité supérieure de la surépaisseur (12), l'arête (9) étant destinée à exercer une pression au niveau de l'insertion des tendons des fléchisseurs au niveau du calcanéus

8. Semelle orthopédique (11) selon la revendication 7, **caractérisée en ce que** chacune des sections transversales à la direction longitudinale antéro-postérieure, de la surépaisseur (12), a une épaisseur constante ou sensiblement constante sur toute sa largeur, à savoir selon une direction transversale interne-externe

## Patentansprüche

1. Orthopädische Vorrichtung (1) zur Befestigung an einer Innensohle ( 4) eines Schuhs, mit einem Fersenentlastungsmittel (2), das ein oberes Ende zur Ausübung eines Drucks senkrecht über der Verbindung des hinteren Teils des Fußgewölbes und des vorderen Teils der Ferse aufweist, das Fersenentlastungsmittel (2) eine maximale Dicke zwischen 6 und 14 Millimetern hat, seine Shore-A-Härte zwischen 15 und 40 liegt, die Härte bei Raumtemperatur mit einem genormten Shore-A-Durometer gemessen wird, das Fersenentlastungsmittel (2) eine Unterseite (5) umfasst, die dazu bestimmt ist, an der Einlegesohle (4) befestigt zu werden, eine geneigte Oberseite (6) und eine konkave Rückseite (7), die sich vom oberen Ende der Oberseite (6) bis zum Ende der entsprechend angeordneten Unterseite (5) erstreckt, wobei die Rückseite (7) einen spitzen Winkel mit der Unterseite (5) bildet, die Verbindung der geneigten oberen Fläche (6) und der hinteren Fläche (7) eine Kante (9) bildet, die dem oberen Ende des Fersenentlastungsmittels (2) entspricht, wobei die Kante (9) dazu bestimmt ist, Druck auf den Ansatz der Sehnen der Beuger am Fersenbein auszuüben.

2. Orthopädische Vorrichtung (1) nach Anspruch 1, **dadurch gekennzeichnet, dass** jeder der Querschnitte quer zur anterior-posterioren Längsrichtung des Fersenentlastungsmittels (2) eine konstante oder im Wesentlichen konstante Dicke über seine gesamte Breite, d.h. in einer Innen-Außen-Querrichtung, aufweist.

3. Orthopädische Vorrichtung (1) nach Anspruch 1 oder 2, **dadurch gekennzeichnet, dass** das obere Ende des Fersenentlastungsmittels (2) eine konkave Form hat, die dazu bestimmt ist, sich an den konvexen Teil des vorderen Teils der Ferse anzupassen.

4. Orthopädische Vorrichtung (1) nach einem der vorhergehenden Ansprüche, **dadurch gekennzeichnet, dass** die maximale Dicke des Fersenentlastungsmittels (2) zwischen 8 und 12 Millimetern liegt.

5. Orthopädische Vorrichtung (1) nach einem der vorhergehenden Ansprüche, **dadurch gekennzeichnet, dass** sie ein Mittel zur Positionierung des Fersenentlastungsmittels (2) umfasst, das sich über das hintere Ende des Fersenentlastungsmittels (2) hinaus erstreckt und dessen hinteres Ende des Positionierungsmittels dazu bestimmt ist, am hinteren Ende der Einlegesohle (4) positioniert zu werden.

6. Orthopädische Vorrichtung (1) nach dem vorhergehenden Anspruch, **dadurch gekennzeichnet, dass** das Positionierungsmittel eine flexible Zunge (15) ist, die nicht dauerhaft mit dem oberen Teil des Fersenentlastungsmittels (2) verbunden ist.

7. Orthopädische Einlegesohle (11) mit einem vorderen Abschnitt (13), einer Verdickung (12) und einem hinteren Abschnitt (14), wobei der vordere Abschnitt (13) dazu bestimmt ist, den Vorfuß und den vorderen Teil des Fußgewölbes zu stützen, und der hintere Abschnitt (14) zur Abstützung der Ferse bestimmt ist, die Verdickung (12) ein oberes Ende umfasst, das dazu bestimmt ist, lotrecht über der Verbindung des hinteren Teils des Fußgewölbes und des vorderen Teils der Ferse Druck auszuüben, die Verdickung (12) eine maximale Dicke zwischen 6 und 14 Millimetern und eine Shore-Härte A zwischen 15 und 40 hat, die Härte bei Raumtemperatur mit einem genormten Shore-A-Durometer gemessen wird, wobei die Verdickung (12) eine geneigte Oberseite (6) und eine konkave Rückseite (7) umfasst, die sich vom oberen Ende der Oberseite (6) bis zum hinteren Abschnitt (14) bzw. vorderen Abschnitt (13) erstrecken, wobei die Verbindung der geneigten oberen Fläche (6) und der hinteren Fläche (7) eine Kante (9) bildet, die dem oberen Ende der Verdickung (12) entspricht, wobei die Kante (9) dazu bestimmt ist, Druck an der Insertion der Sehnen der Beuger am Fersenbein auszuüben.

8. Orthopädische Einlegesohle (11) nach Anspruch 7, **dadurch gekennzeichnet, dass** jeder der Querschnitte quer zur anterior-posterioren Längsrichtung der Verdickung (12) eine konstante oder im Wesentlichen konstante Dicke über ihre gesamte Breite aufweist, und zwar in einer Innen-Außen-Querschnittsdiktion.

## Claims

1. An orthopaedic device (1) for attachment to an insole (4) of a shoe, comprising a heel relief means (2) which comprises an upper end for exerting pressure in line with the junction of the posterior part of the plantar arch and the anterior part of the heel, the heel relief means (2) having a maximum thickness of between 6 and 14 millimeters, its shore A hardness being between 15 and 40, the hardness being measured at ambient temperature using a standardized shore A Durometer, the heel relief means (2) comprising a lower face (5) intended to be fixed to the insole (4), an inclined upper face (6) and a concave rear face (7) extending from the upper end of the upper face (6) to the end of the correspondingly arranged lower face (5), the rear face (7) forming an acute angle with the lower face (5), the junction of the inclined upper face (6) and the posterior face (7) forming a ridge (9) corresponding to the upper end of the heel relief means (2), the ridge (9) being intended to exert pressure at the insertion of the flexor tendons at the calcaneus.

2. Orthopedic device (1) according to claim 1, **characterized in that** each of the sections transverse to the longitudinal antero-posterior direction of the heel relief means (2) has a constant or substantially constant thickness over its entire width, namely in an internal-external transverse direction.

3. Orthopaedic device (1) according to claim 1 or 2, **characterized in that** the upper end of the heel relief means (2) has a concave shape intended to match the convex part of the anterior part of the heel.

4. Orthopaedic device (1) according to any one of the preceding claims, **characterized in that** the maximum thickness of the heel relief means (2) is between 8 and 12 millimetres.

5. Orthopedic device (1) according to any of the preceding claims, **characterized in that** it comprises a means for positioning the heel release means (2), extending beyond the rear end of the heel release means (2) and the rear end of which is intended to come into position at the rear end of the insole (4).

6. Orthopedic device (1) according to the preceding claim, **characterized in that** the positioning means is a flexible tongue (15) non-permanently connected to the upper part of the heel relief means (2).

7. Orthopedic insole (11) comprising a front portion (13), a thickened portion (12) and a rear portion (14), the front portion (13) being designed to support the forefoot and the front part of the plantar arch, the rear portion (14) being designed to support the heel, the thickening (12) comprising an upper end designed to exert pressure in line with the junction of the posterior part of the arch of the foot and the anterior part of the heel, the thickening (12) has a maximum thickness of between 6 and 14 millimeters and a shore A hardness of between 15 and 40, the hardness is measured at room temperature using a standardized shore A Durometer, the allowance (12) comprising an inclined upper face (6) and a concave rear face (7) extending from the upper end of the upper face (6) to the rear portion (14) or the front portion (13) respectively, the junction of the inclined upper face (6) and the posterior face (7) forming a ridge (9) corresponding to the upper end of the thickening (12), the ridge (9) being intended to exert pressure at the insertion of the flexor tendons in the calcaneus.

8. Orthopedic sole (11) according to claim 7, **characterized in that** each of the sections of the thickening (12) transverse to the longitudinal antero-posterior direction has a constant or substantially constant thickness over its entire width, i.e. in an internal-external transverse direction.
